# EUROPEAN PATENT APPLICATION

(11) **EP 1 496 363 A1**
(43) Date of publication of application: **12.01.2005**
(21) Application number: 03712842.8
(22) Date of filing: 24.03.2003
(51) Int. Cl.: G01N 33/543, G01N 33/545, G01N 33/553

(54) **BIOCHIP SENSOR SURFACE CARRYING POLYETHYLENE GLYCOLATED NANOPARTICLES**

(30) Priority: 03.04.2002 JP 2002101134
(71) Applicant: Japan Science and Technology Agency, Kawaguchi-shi, Saitama 332-0012 (JP)
(72) Inventor: KATAOKA, Kazunori, Nakano-ku, Tokyo 165-0031 (JP); NAGASAKI, Yukio, Moriya-shi, Ibaraki 302-0128 (JP); OTSUKA, Hidenori, Tsukuba-shi, Ibaraki 305-0044 (JP); UCHIDA, Katsumi, Kashiwa-shi, Chiba 277-0005 (JP); ISHII, Takehiko, Kitakatsushika-gun, Saitama 340-0211 (JP); SUZUKI, Yuko, Furukawa-shi, Miyagi 989-6151 (JP); AKIYAMA, Yoshitsugu, Kita-ku, Tokyo 114-0014 (JP); TAKAE, Seiji, Tama-shi, Tokyo 206-0033 (JP)
(74) Representative: Albrecht, Thomas, Dr.
(86) International application number: PCT/JP2003/003504
(87) International publication number: WO 2003/083478

(57) **Abstract**

The invention provides high sensitivity bioassay sensor systems in which non-specific adsorption of impurities such as, for example, proteins, in biological samples is inhibited. Polyethylene glycolated particles enclosing metal or semi-conductor which is in common with the sensor material are used for amplification.

## Description

### Technical Field

This invention relates to technical field of bioassay. More specifically, the invention relates to a biosensor system wherein non-specific adsorption or binding of impurities other than an analyte contained in biological fluids or the like is reduced or prevented, or the analyte-detecting sensitivity can be increased; and also to an assay method using said biosensor system.

### Background Art

As a means for detecting analytes present in biological samples, biosensors having a large variety of detection systems have been proposed. Of such biosensors, sensors utilizing surface plasmon resonance (SPR) are sensitive to changes in refractive index at and near the surface of a metal film (e.g., see A. Szabo, et al., Curr. Opin. Strnct. Biol., 5 (1995) 699 - 705). SPR allows in situ observation of procedures taking place between the surface and a complex biological solution and renders available real time analyte data, without the use of, e.g., a marker. Hence SPR is suitable for collection of kinetic and thermodynamic parameters, and SPR-utilizing sensors are one of those drawing keen attention nowadays.

As a typical biosensor chip having such a surface, BIACORE® available from Amersham Pharmacia Biotech., Inc. can be named. In BIACORE® , semi-transparent matrix of dextran with carboxylated end is immobilized on a thin gold membrane. More specifically, it provides a biosensor chip formed by the steps of: linking organic molecules expressed by a formula HS-R-Y (wherein R stands for a hydrocarbon chain having a chain length exceeding ten atoms and which may be interrupted with hetero atom(s), and Y stands for a ligand or an active group for covalently bonding a biocompatible porous matrix thereto) onto a thin membrane surface of a free electron metal such as gold, silver or the like via the thiol (or mercapto) groups therein, whereby covering said surface with a close-packed monolayer thereof, and thereafter covalently bonding to the surface hydrogel as said biocompatible porous matrix, said hydrogel comprising agarose, dextran, polyethylene glycol and the like which may have functional group(s) for linking to the ligand (see, e.g., U. S. Patent 5,763,191). In occasions of detecting a biological substance such as protein on such a biosensor chip, a fixed amplification of SPR signal and prevention of non-specific adsorption are achieved.

Also mainly for the purpose of preventing non-specific adsorption of impurities which are present in biological fluids or the like onto sensor chip surfaces, in occasions of quantifying intended analyte proteins or the like, there has been provided a sensor ship having a surface formed of a spacer molecule (C₁ - C₃₀ alkylene chain) which links onto the support via a sulfur atom (of mercapto group) and to which covalently bonded are, by order, a hydrophilic linker (a straight chain molecule of 4 to 15 atoms in chain length) and a solid phase reactant (biotin derivative residue) (see, e.g., U. S. Patent 3,071,823). Also provided are sensor chips having a self-assembled monolayer linked onto a golden surface via mercapto groups, using a compound based on HS-spacer molecule (C₁₁ alkylene chain)-hydrophilic linker ( a chain composed of 3 or 6 ethylene oxide units) (see for example, Roberts et al., J. Am Chem. Soc., 1998, 120, 6548 - 6555). Still another proposal was made for a sensor chip having a surface carrying heterotelechelic polymer whose ethylene oxide units are within a range of 5 - 10,000 (see WO 01/86301 A1).

As already stated, sensor chips having such surfaces as above-described could accomplish a fixed amplification of SPR signals. Whereas, as biosensor systems capable of further increasing detection sensitivity, many which use colloidal gold (Au) in combination with sensor chips carrying thin gold membrane on their surfaces (which do not carry such a dextran layer or polyethylene glycol layer as described in the literature references as above-cited) have also been proposed. Compared with the systems not using colloidal gold, said systems in general exhibit the advantages of achieving large shifts in plasmon angles, broad width plasmon resonance and remarkable increase in the minimum reflectivity (e.g., see L. A. Lyon et al., Anal. Chem., 1998, 70, 5177 - 5183, in particular, "Introduction" at page 5177; J. Am. Chem. Soc., 2000, 122, 9071 - 9077; E. Hutter et al., J. Phys. Chem. B 2001, 105, 11159 - 11168; JP 2002-267669A; and JP 2000·55920A). It is in common among those known sensor chips of the system using colloidal gold or gold nanoparticles for amplification or enhancenent of SPR, that surfaces of their thin gold membranes are modified with alkanethiol (e.g., 3-mercaptopropionic acid or 3-mercaptoethylamine) or the like, and to which biotin, avidin or streptavidin, antibody, or the like are covalently bonded. Colloidal gold or gold nanoparticles are bound to (including chemical adsorption) proteins using or without using alkanethiol as referred to in the above, to form biologically specific binding pair such as biotin-streptavidin, antigen-antibody and the like, and whereby linked onto said sensor chip surfaces. Furthermore, E. Hutter et al. suggests: when gold nanoparticles are directly immobilized on gold or silver support (sensor chips) using 2-aminoethanethiol (AET) or 1,6-hexanedithiol (HDT), the Au/AET/Au system exhibits enhanced SPR sensitivity, while Au/HDT/Au system shows a considerably lower amplification effect of the gold nanoparticles (see p. 11159, "Introduction"). It is also known that interaction between biological molecules can be pursued on real time basis on glass sensor chip surfaces with visible-UV spectrophotometer, where a self-assembled monolayer is formed on said chips using such gold nanoparticles (see, for example, N. Nath et al., Anal. Chem., 2002, 74, 504 - 509).

Separately from construction of biosensor systems as above, there has been proposed a method of improving dispersion stability of metal particles which are used as a marker in bioassays, by modifying surfaces of said metal particles with polymer chain such as polyethylene glycol (or polyethylene oxide) which is water-soluble and has high mobility in aqueous media (W. Pwuelfine et al., J. Am. Chem. Soc., 120(48), 12696 - 12697 (1998)). Polyethylene glycolated (PEG-modified) metal particles, semi-conductor particles or magnetic particles in which polyethylene glycol linked the metal particle surface has a functional compound residue at one other than the one linked to said surface are also known to exhibit dispersion stability in aqueous media (see Otsuka et al., J. Am. Chem. Soc., 2001, 123, 8226 - 8230); JP 2001-200050A; JP 2002-80903A). Use of semiconductor nanoparticles surrounded by a polymer (e.g., diacetylene, styrene or the like )as a probe for detecting biological substances has also been proposed (see, for example, USP 6,207,392).

In the aforesaid systems using gold nanoparticles for improving sensitivity of SPR, it has been suggested that the sensitizing effect differs depending on the distance between the gold nanoparticles and the thin gold membrane surfaces of the biosensor chips or on the linkage mode of said particles with said surfaces (see, for example, above-cited N. Nath et al.). Therefore, even when such a system using gold nanoparticles and biosensor chips as above-described is applied to the technology disclosed in U. S. Patent 5,763,191, there exists a probability that either sensitivity of the system is reduced or non-specific adsorption of impurities cannot be prevented.

### Disclosure of the Invention

We have discovered that combined use of BIACORE® sensor chips or those having polyethylene glycol-modified surfaces with PEG-modified metal particles or semiconductor particles which have been provided mainly for improving dispersion stability in aqueous media could increase bioassay sensitivity with the corresponding sensor chips and at the same time could prevent or control non-specific adsorption of impurities. The present invention is completed based on this knowledge.

According to the invention, a biosensor system for bioassay is provided, which comprises, as a set,
(A) polyethylene glycol-modified nanoparticles of a structural formula I:

   (X-W²-PEG-W¹-L)ₓ-PCL-(L-W¹-PEG-W²-Y)_{y} (I)

   (in which
   PCL stands for a free electron metal fine particle, metal oxide fine particle or semiconductor fine particle;
   X stands for a functional group or functional moiety capable of linking to a biosensor chip surface;
   Y stands for at least one group or moiety which is selected from the group consisting of C₁-C₆ alkyl, optionally protected functional groups which are useful for forming said functional group or functional moiety X, and functional moieties same as , or different from, X;
   L stands for a linker group or moiety bound to PCL;
   W¹ and W² stand for single bonds or same or different linkers
   PEG stands for ethylene oxide units, (-CH₂CH₂O-)ₙ (wherein n is a integer of 5 - 10,000),
   W²-PEG-W¹-L in (X-W²-PEG-W¹-L)ₓ and
      (L-W¹-PEG-W²-Y)_{y} may be same or different, and
   x and y are integers not less than 1 independently of each other, which together represent an integer sufficient for the PEG chains to cover the PCL surface in an aqueous medium; and
(B) a biosensor chip having a surface to which above (A) particles can be linked via X and which surface is made of dielectrics such as glass or a material corresponding to that of PCL.

As another embodiment of the present invention, there are provided polyethylene glycol-modified nanoparticles wherein X in said structural formula I is a residue of a member constituting a biological specific binding pair; Y is a group other than the residue constituting the biological specific binding pair; L stands for a group expressed by a formula, 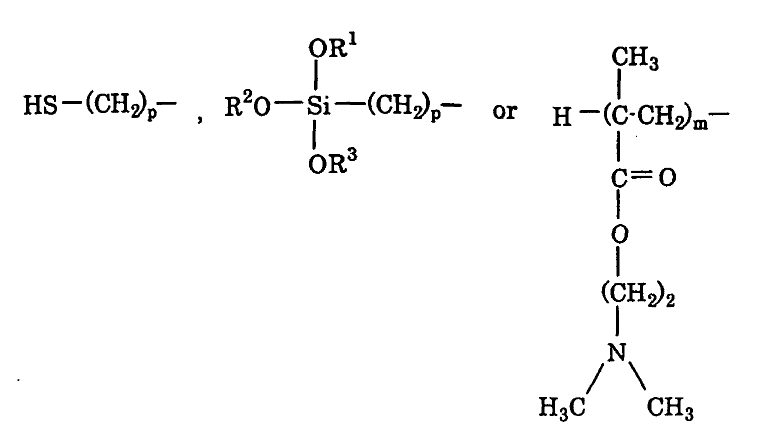 (in which p is an integer of 2 - 12, R¹, R² and R³ each independently stands for C₁-C₆ alkyl, and m is an integer of 2 - 100);
x + y is a number corresponding to 0.1 - 0.5, preferably 0.25 - 0.40, per 1 nm² of the PCL surface; (x/ x + y) × 100 is an integer of 1 - 99, preferably 20 - 65; and the average size of cross-section of the PCL is within a range 1 - 500 nm, preferably 5 - 500 nm.

The invention furthermore provides a method of detecting an analyte in a biological fluid, which comprises:
(a) preparing above-described polyethylene glycol-modified nanoparticles,
(b) preparing a biosensor chip having a thin membrane surface made of a material corresponding to that forming PCL of the nanoparticles, said surface carrying, either directly or via at least a C₁-C₆ alkylene or (-CH₂CH₂O-)ₙ (wherein n is an integer of 5 - 10,000), a member which is to form a biological specific binding pair with the other member of the pair which is present in X of said (a) nanoparticles,
(c) contacting said particles (a) and biosensor chip (b) with a biological fluid which is suspected to contain either one of the members capable of forming the biological specific binding pair as an analyte,
(d) determining the change in the extent of linkage of the particles (a) and the biosensor chip (b) surface caused by the competitive action of the analyte, and
(e) using the change as an index of the analyte concentration in said biological fluid.

The (A) particles-(B) biosensor chip set following the present invention, when they are linked covalently or non-covalently (e.g., hydrophobic bond, ionic bond, chemical adsorption and the like that are seen in biological specific binding), increases for example resonance Raman scattering by surface sensitizing effect.

In particular, with fine particles of free electron metal, notable changes in surface plasmon resonance signals (large shift in plasmon angle, broad width plasmon resonance and increase in the minimum reflectivity) are brought about. Surprisingly, such changes are recognized also when BIACORE® sensor chip, a biosensor chip carrying a dextran layer of a considerable thickness, is linked to said (A) particles.

Furthermore, said (B) biosensor chip with its surface coated with (A) particles following the present invention can, even when said chip surface were not coated with such a dextran layer or not polyethylene glycol-modified, significantly suppress non-specific adsorption of, for example, protein present in biological fluids.

### Brief Explanation of Drawings

Fig 1 is a schematic illustration showing the relationship between PEG-modified gold nanoparticles and the sensor chip surface following the present invention, in which a) is a schematic view of the high sensitivity system comprising a metal surface onto which PEG-modified gold nanoparticles are immobilized, (i) standing for PEG chains inhibiting non-specific adsorption, (ii) standing for ligand molecules, and (iii) standing for gold particles strengthening SPR response; and b) illustrates construction of the competitive assay system.

Fig. 2 is a sensorgram showing the result of an experiment conducted for confirming specific binding of lac 65 and lectin.

Fig. 3 are graphs showing the relationship between lactose density on the PEG-modified gold nanoparticle surfaces and response of the lectin-immobilized surface.

Fig. 4 are sensorgrams illustrating dissociation of a PEG-modified gold nanoparticles and a sensor chip which are bound via lactose-lectin, caused by competition with galactose.

Fig. 5 is a graph showing the measured result of zeta-potential of a PEG-modified gold nanoparticles, said PEG having amino groups at their unbound ends (•-marked curve) and similar measurement result of gold nanoparticles having acetalized formyl groups at unbound ends (■-marked curve).

Fig. 6 is a graph showing a quantitative measurement result of protein carried by PEG-modified gold nanoparticles having biotin residues at the unbound ends.

Fig. 7 is a graph showing adsorbability of various proteins onto a gold chip surface to which PEG-modified gold nanoparticles having amino groups at their unbound ends have been directly adsorbed.

Fig 8 are graphs showing protein adsorbability of the gold chip surface onto which the gold nanoparticles which are used in Fig. 7 are linked utilizing N-succinimidyl-3-(2-pyridylthio) propionate (SPDP).

### Disclosure of the Invention

While the usage which draws our particular attention of the biosensor systems according to the present invention is for a bioassay (assay of biological molecules) utilizing surface plasmon resonance (SPR), the term, assay, as herein used includes assays which utilize changes in traceable signals other than SPR, radioactivity, contact angle of various electromagnetic waves, sedimentation, ultraviolet spectrum, Raman scattering and the like. Biological molecules which are the object of detection by bioassays intended by the invention may be one of the constituents of a "biological" specific binding pair (e.g., those formed by hydrophobic binding, ionic binding or the like of biological molecules), more specifically, either one of the constituents of non-covalently bound pair such as a ligand and receptor, for example, antigen or hapten and antibody, sugar and lectin, substrate and enzyme, hormone and receptor thereof, oligonucleotide and complementary chain thereof, biotin and avidin or streptavidins, etc., while not limited to the foregoing.

"Biosensor systems" said in this invention signify each of elements, their assemblies or combinations that are useful for conducting above-described assays. Furthermore, in the present specification the terms, "fine particles" and "nanoparticles", are exchangeably used and, unless otherwise specified, include those of the size orders ranging from sub-nanometers to several micrometers, not limited to nanometer size particles.

Hereinafter construction of the present invention is described in detail.

### (A) Re. PEG-modified nanoparticles represented by the structural formula I:

PCL can be fine particles of a material selected from a group consisting of free electron metals (e.g., gold, silver, platinum, aluminum, copper and the like), semiconductors (e.g., CdS, ZnS, CdSe, InAs and the like) and metal oxides (e.g., TiO₄, Cr₂O₃ and the like). Those particles having an average cross-sectional size ranging 1- 500 nm can be conveniently utilized while not limited thereto.

L stands for a linkage to said particle surface, via a group or moiety which is capable of linking to said surface (e.g., by chemical binding or chemical adsorption, or covalent bonding via surface -OH group formed by hydroxylation where the particle is made of metal oxide), which may be any so long as it meets the purpose of the present invention. Whereas, preferably it is a linkage via a linker selected from those of the following formulae (i), (ii) and (iii): 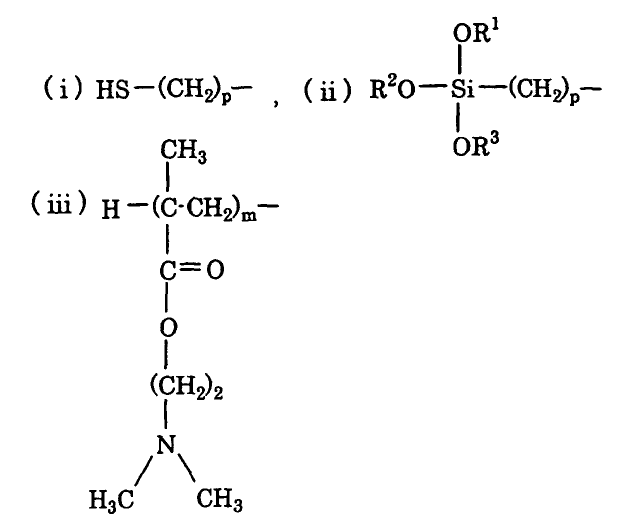 and (in which p is an integer of 2 - 12; R¹, R² and R³ each independently stands for C₁-C₆ alkyl; and m is an integer of 2 - 500, preferably 5 - 100). Such a linkage can be one formed with said particle surface where the linker or moiety (segment) of above formula (i) or (iii) is selected. Where a linker or moiety of the formula (ii) is selected, the linkage may be one formed with dealcoholizing reaction between -OH on the hydroxylated metal oxide surface and silanol group.

PEG stands for ethylene oxide units: (-CH₂CH₂O-)ₙ (where n is an integer of 5 - 10,000, preferably 10 - 10,000, more preferably 20 - 2,500).

X represents a functional group or functional moiety which is capable of linking to the biosensor chip surface. Said functional group or functional moiety may be selected from those expressed by the above formulae (i), (ii) and (iii) which are given as examples of L, or they may be residues of one of the constituents forming aforesaid biological specific binding pair , or residues of proteins which do not affect intended bioassays.

Of such constituents of specific binding pairs, generally those of low molecular weight, e.g., residues derived from hapten, sugar, substrate, hormone, oligonucleotide and biotin are preferred.

Y can be a C₁-C₆alkyl, a group or functional moiety as defined as to X which moiety being optionally protected, or an optionally protected group or functional moiety differing from X. As the typical of such groups or moieties differing from X, those selected from the groups of the following formulae (iv), (v) and (vi): 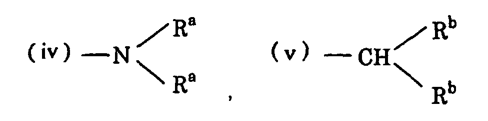 and

( vi ) ―COOH

(in which R^{a} each independently stands for hydrogen or C₁-C₆ alkyl; R^{b} each independently stands for a C₁-C₆ alkyloxy: or the two R^{b}'s together stand for an atomic group forming an optionally oxy- or C₁-C₆ alkyl-substituted ethylene group) can be named.

As preferred Y, a group or moiety selected from the group consisting of those expressed by above formulae (iv), (v) and (vi), those of the formula (i) as defined as to X, and C₁-C₆ alkyl can be named.

W¹ and W² each independently can be a group selected from the group consisting of linkers, e.g., single bond; C₁-C₆ alkylene, -COO- (binding to methylene group in an ethylene oxide unit via oxygen atom), -O-, -S-, -(C₁-C₆alkylene)-COO-, -(C₁-C₆ alkylene)-O- and -(C₁-C₆ alkylene)-S-.

The polymers represented by the formulae (II) and (III) which are composed of the foregoing linkers, moieties and /or segments:

(II) X-W²-PEG-W¹-L,

and

(III) L-W¹-PEG-W²-Y

may be the same , as can be understood from the above definitions of X and Y, but preferably they are different. Again, W²-PEG-W¹-L in these formulae may be the same or different. One or more whole numbers (corresponding to said integers x and y, respectively and independently of each other) of the polymers of the formulae (II) and (III) bind to single PCL surface. The sum of x + y is an integer sufficient for the PEG chains to cover the PCL surface, which number should be such that allows a polyethylene glycolated particle surface (when such particles cover a sensor chip surface, so covered chip surface) to suppress non-specific adsorption of protein or the like thereonto in an aqueous medium. As for the extent of suppression, later appearing Examples can be used for reference. "Non-specific adsorption of protein or the like" signifies adsorption other than that occurring through specific binding, e.g., where X is a member capable of forming a biological specific binding pair, for example, an antigen, its binding to an antibody corresponding thereto. Although not in any limitative sense, x + y can be such that will make the polymer chain number 0.1 - 0.5, preferably 0.25 - 0.40, per 1 nm² of the PCL surface to which they bind. The ratio between x and y is optional, as the polymers expressed by the formulae (II) and (III), respectively, may be the same as aforesaid. Whereas, in assays which utilize competitive actions of analytes to biological specific binding of biosensor surfaces with PEG-modified particles as later described, the ratio of x to the total number of x + y can range 1 - 99, preferably 20 - 65. A polymer of the formula (II) (in which X is one of the constituents of a biological specific binding pair) and a polymer of the formula (III) (in which Y is different from X and is a group or moiety not binding with X) can be disposed on PCL at a ratio within the above-specified range. The particles following such embodiments are useful for conducting speedy and highly sensitive assays.

The typical of such polyethylene glycolated particles are disclosed in aforesaid Otsuka te al., J. Am. Chem. Soc., 2001, 123, 8226 - 8230, JP 2001-200050 A and JP 2002-80903A, and also can be prepared following the descriptions therein. Their disclosures can also be utilized for forming said particles. In particular, the heterotelecheric polymers can be easily made by skilled artisans, referring to functionalization of α, ω-terminals of block copolymers as described in WO 96/32434, WO 96/33233 and WO 97/06202, which have been proposed by a part of the present inventors.

In the definitions given above, the terms, C₁-C₆ alkyl, C₁-C₆ alkyloxy or C₁-C₆ alkylene, have common significations even when they are used as to different groups or moieties. For example, C₁-C₆ alkyl may be methyl, ethyl, n-propyl, iso-propyl, n-butyl, sec-butyl, n-hexyl or the like, and as C₁-C₆ alkyloxy, alkyloxy groups corresponding to above-named C₁-C₆ alkyl can be named. Also C₁-C₆ alkylene includes methylene, ethylene, propylene, 1, 3-trimethylene, 1, 6-hexamethylene and the like.

### (B) Re. biosensor chips

The shape and dimension of biosensor chips used in this invention are not critical, so long as their surfaces are capable of linking to the X groups or moieties of the PEG-modified nanoparticles which are fully described in (A) above, and can be utilized for bioassays. Preferably, however, the surface is formed of the same material or a material belonging to the same class, to that forming the PCL used in a set therewith (e.g., gold and gold, gold and silver, where free electron metal(s) are used; CdS and CdS, CdS and InAs, where semi-conductor(s) are used). This is convenient for enhancing the signals obtained from the (A) particles and the sensor chips as earlier described. Whereas, when the signals are detected with visible-UV spectrometer, the surface may be made of crystal or glass which can transmit these lights. The surface may normally be a thin membrane formed by vacuum vapor deposition of corresponding material.

Such a surface may be either so modified as to promote formation of linkages with X groups or moieties on said (A) particles or, when X is protein, may be of unmodified material per se forming the chip surface. The modification can be made with organic compound as described as to L in the formula (I), which has a group or moiety bindable to PCL on at least one of their ends. For example, with the chips having surfaces made of gold, silver or semi-conductor, the surfaces can be modified with alkanethiol (e.g., 3-mercaptopropionic acid or 2-mercaptoamine) and then covalently bonded with a member capable of forming a biological specific binding pair with the member X on the corresponding PEG-modified nanoparticles, utilizing free carboxyl or amino, to complete desired surfaces. Examples of biosensor chips having such surfaces are described in aforesaid L. A. Lyon et al., E. Hutter et al., JP 2002-267669A and JP 2000-55920A.

Besides, BIACORE® sensor chips carrying a dextran layer on their surfaces, or sensor chips with surfaces covered with heterotelecheric polymer having poly (oxyethylene) chain in the middle (e.g., see WO 01/86301 A1) can also be used in the present invention.

Skilled persons in the art will be able to prepare still other sensor chips, by referring to the foregoing conventional technologies.

Typical heterotelecheric polymers useful for modifying the above-described PCL surfaces and sensor chip surfaces can be made according to the following reaction schemes. 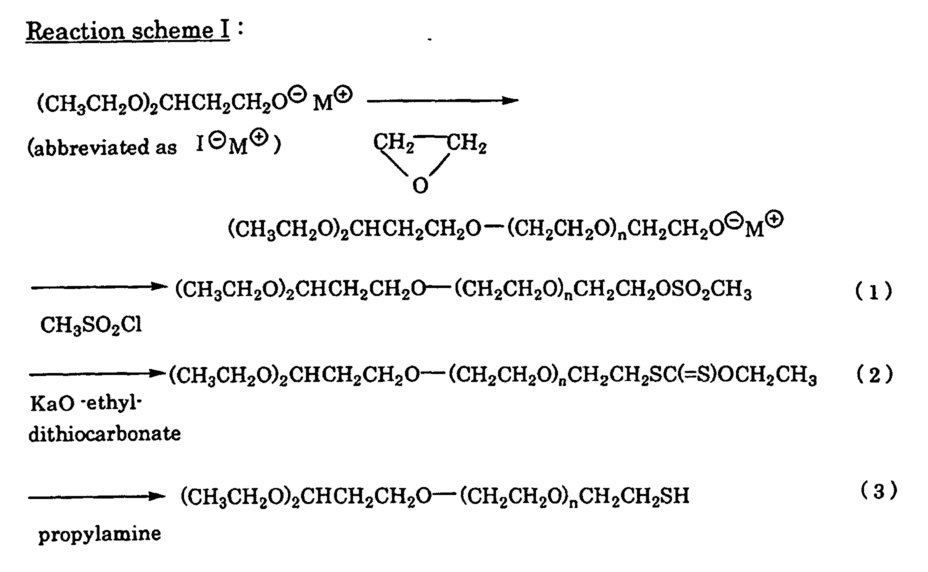 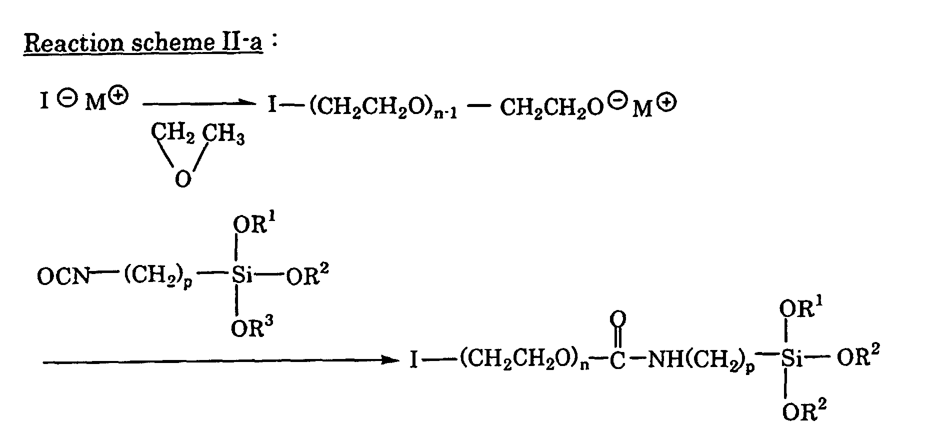 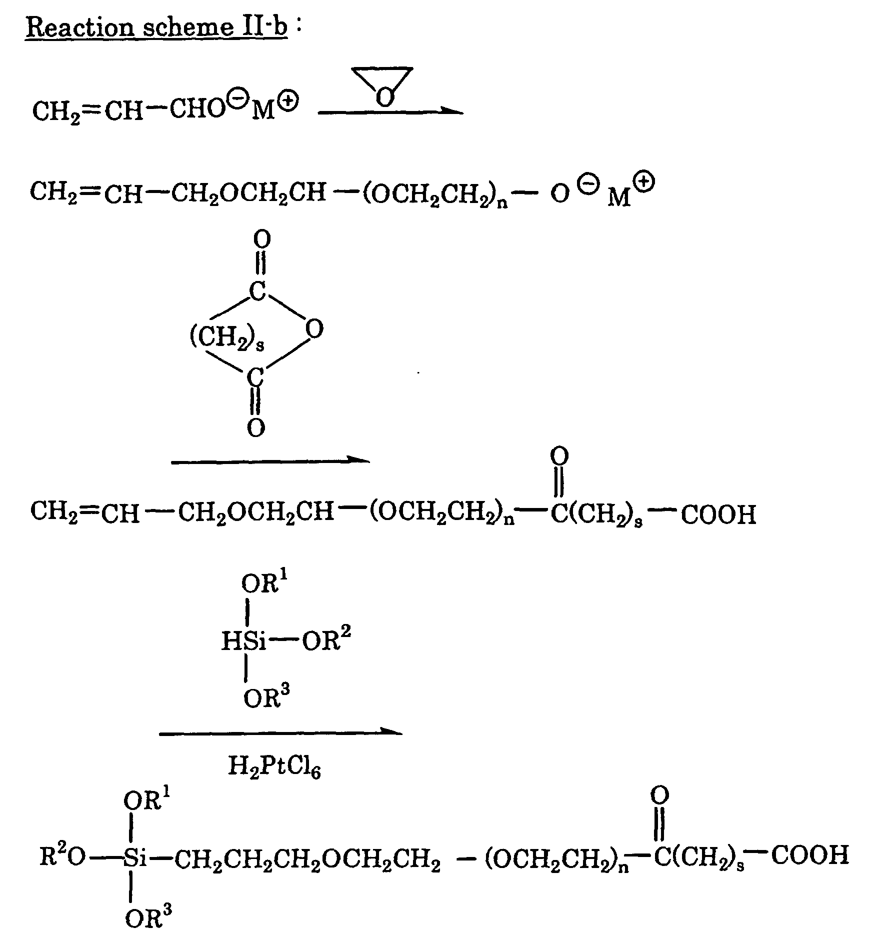 (in the above formulae, M stands for potassium, sodium or lithium).

The foregoing living polymerization steps can be conducted under the reaction conditions known per se ( for example, see said WO 96/32434, WO 97/06202, etc.), or under those following later appearing Examples or modifying the given conditions.

Also a polymer of the formula, 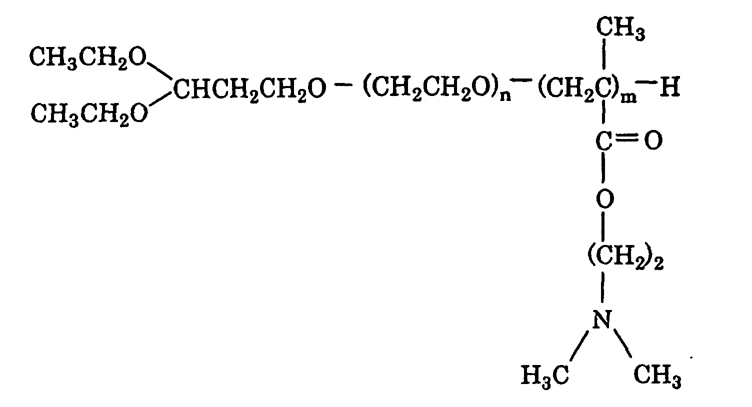 is obtainable following the method as described in Kstaoka et al., Macromolecules, 1999, 32, 6892 - 6894, a thesis reported by a part of the present inventors. A copolymer of PEG Mw=5000 g/mol and PAMA (poly [2-N, N-dimethylamino] ethyl methacrylate]) having a degree of polymerization m=68) was used in the later described PEG-modified fine particles.

PEG-modification of PCL using these polymers shall be briefly explained. Fine particles of free electron metals, metal oxides or semi-conductors which constitute PCL in the structural formula I may be those available in the market or may be obtained by preparing corresponding colloids. Also those PEG-modified nanoparticles according to the present invention may be prepared by causing concurrent presence of above polymer precursor in the step of forming corresponding fine particles (having average particle size of , for example, 0.5 nm - 1 µm, preferably 1 nm - 200 nm), whereby providing a surface as expressed by the structural formula I onto which the polymer precursor is linked to the fine particle surface, or a precursor surface thereof.

### Re. (A) particles (B) sensor chip set

Said (A) particles and (B) sensor chip are used in such a manner that the surface coming into contact with a sample fluid suspected of containing an object analyte is to be substantially covered by the (A) particles, in the cases other than those wherein (B) sensor chip surfaces have a dextran layer, like BIACORE® or are modified with polymers having poly (ethylene oxide) chains in the middle as described in WO 01/86301 A1. In particular, such (A) particles and (B) sensor chip are used in linked state. The (B) sensor chip surface which is covered with (A) particles can significantly suppress non-specific adsorption of protein or the like onto said surface, by the action of the polymer(s) of the formulae (II) and (III) which are on the (A) particle surfaces. Obviously, amplification effect of various signals by the particles is also achieved.

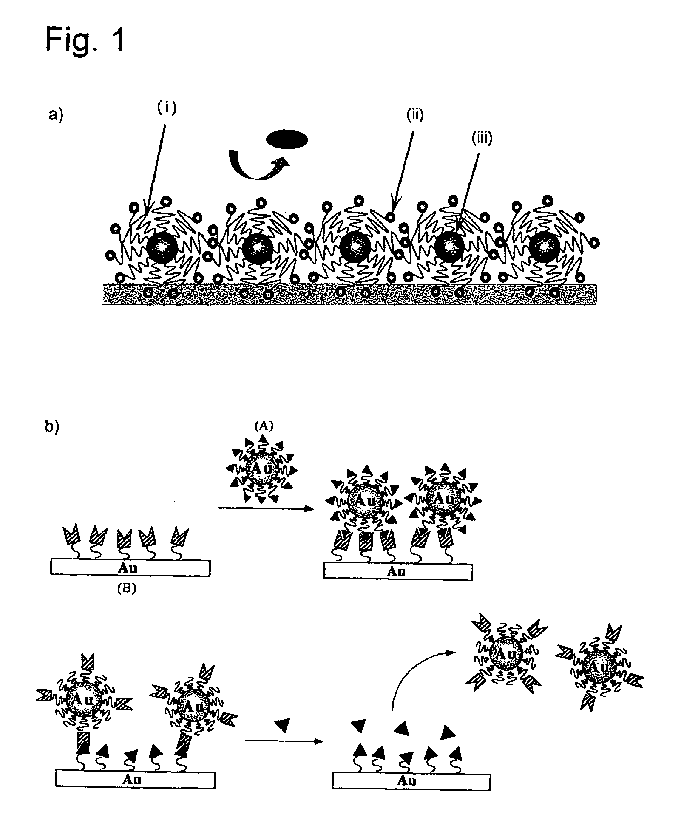

Where X in the (A) particles is one of the members to form a biological specific binding pair and the other member of the pair is present on the (B) sensor chip surface, said (A) particles and the (B) sensor chip can be used as a set in an embodiment as illustrated in Fig. 1 which schematically shows the concept of an assay. In said figure, the triangles represent, for example, sugar, biotin, antigen or hapten, hormone, oligonucleotide or the like and the marks into which the triangles fit represent lectin, avidin or streptavidin, antibody, receptor protein, complementary oligonucleotide or polynucleotide containing said nucleotide sequence, or the like. The wavy lines linked to these marks can be poly (ethylene oxide) segments.

A bioassay method illustrated by such schematic views also is an embodiment of the present invention.

That is, the invention provides a detection method of an analyte in a biological fluid, which comprises, in general,
(a) preparing (A) particles which can be made according to the foregoing descriptions,
(b) preparing a biosensor chip having a thin membrane surface made of a material corresponding to that forming PCL of the nanoparticles, said surface carrying, either directly or via at least a C₁-C₆ alkylene or (-CH₂CH₂O-)ₙ (wherein n is an integer of 5 - 10,000), a member which is to form a biological specific binding pair with the other member of the pair present in X of said (A) nanoparticles,
(c) contacting said particles (a) and biosensor chip (b) with a biological fluid which is suspected to contain either one of the members capable of forming the biological specific binding pair as an analyte,
(d) determining the change in the extent of linkage of the particles (a) to the biosensor chip (b) surface caused by the competitive action of the analyte, and
(e) using the change as an index of the analyte concentration in said biological fluid.

The changes in the extent of linkage between the (a) particles and (b) sensor chip in above step (d) preferably take the form of changes in surface plasmon resonance spectrum, e.g., shift in plasmon angle or increase in the minimum reflectivity.

Theoretically, such an assay method is applicable to any aqueous fluid samples suspected of containing a member (analyte) capable of constituting a biological specific binding pair, while it is particularly intended for application to biological fluids, e.g., serum, plasma, urine, saliva, and the like, or their concentrates or dilutions.

The method allows speedy and high sensitivity assays.

Hereinafter the present invention is explained more specifically, referring to working Examples, it being understood that the invention is in no way thereby limited.

### Production Example 1:

Preparation of PEG-modified gold fine particles (1)
Polymer used: Acetal-PEG-SH (Mn=5,000)

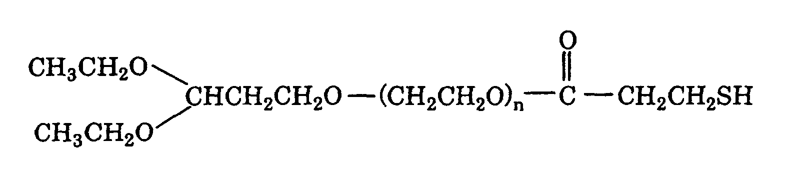

To an aqueous solution of acetal-PEG-SH: HAuCl₄ = 1/6:1 (molar ratio) mixture, ten-fold molar amount to the HAuCl₄ of NaBH₄ was added, and a gold colloid was prepared by reduction process. The end acetal group was treated with pH2 hydrochloric acid and converted to aldehyde group, and the gold colloid was reacted with p-aminophenyl-β-D-lactopyranoside to provide an aqueous solution of lactose-PEG-SH-modified colloidal gold (average particle size: 8.7 nm).

Said acetal-PEG-SH was prepared as follows.

Distilled tetrahydrofuran (THF) 20 ml and 3,3-diethoxy-1-propanol, an initiator, 0.2 mmol (0.032 ml) were added to an argon-substituted reactor, and further an equivalent amount of potassium naphthalene was added, followed by 15 minutes' stirring to conduct metallization. Then ethylene oxide 22.7 mmol (1.135 ml) was added, followed by two days' stirring at room temperature to conduct polymerization. As a reaction-suspending agent, N-succinimidyl-3-(2-piridylthio)propionate (SPDP) 0.4 mmol (0.125 g) was dissolved in a small amount of distilled THF and into the resultant solution said polymerization reaction solution was dropped under cooling with ice, through an isopiestic dropping funnel. After an overnight stirring, the reaction was suspended and the polymer was recovered by the series of operations as washing with saturated saline solution, extraction with chloroform, reprecipitation from ether and lyophilization with benzene. The construction of the recovered polymer was confirmed with ¹H-NMR, and the amount of SPDP residue introduced into the polymer terminals was confirmed by UV absorption of 2-thiopyridone which was released upon reaction with 2-mercaptoethanol.

PEG-SS-Py 2.0×10⁻² mmol (100 mg) was dissolved in 4 ml of distilled water, to which further 5 molar times thereof of dithiothreitol 0.1 mmol (15.42 mg) was added, followed by 30 minutes' stirring at room temperature. After the reaction, the polymer (hereafter abbreviated as PEG 5000) was recovered through a series of operations as washing with saturated saline water, extraction with chloroform and reprecipitation from ether. The construction of the recovered polymer was confirmed with ¹H-NMR and the terminal SH group was quantified by the reaction with 2-pyridyldisulfide (2-PDS).

### Production Example 2:

Preparation of PEG-modified gold fine particles (2)
Polymer used: Acetal-PEG-SH (Mn=3200)

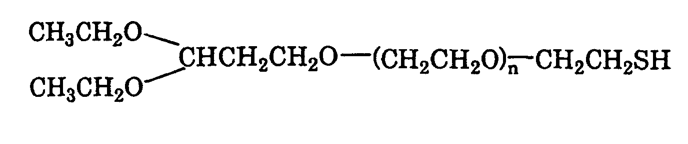

### (1) Preparation of the polymer used

Following the reaction scheme 1, a hetero-bifunctional PEG having acetal group and methylsulfonyl group was synthesized through anionic polymerization, using 3,3-diethoxy-1-propanol as the initiator and methylsulfonyl chloride as the suspender. Further reacting the same with potassium ortho-ethyldithiocarbonate in tetrahydrofuran (THF) at room temperature for 3 hours, a polymer whose methylsulfonyl group was converted to ethyl dithiocarbonate was obtained.

Thereafter, by a further reaction with propylamine again in THF, a hetero-bifunctional PEG (acetal-PEG-SH) expressed by the above formula, which has a mercapto group at α-terminal was obtained.

### (2) PEG-modification of gold particles

Acetal-PEO-SH (Mn=3200) and acetal-PEO-OH (Control) (Mn=3000) were measured out each in an amount as would make the molar ratio of the polymer to gold particles 5.0 × 10⁶:1 and dissolved in 2.0 mL of pure water. Adjusting pH of the solutions to 6.5 with NaOH solution, 1.0 mL of gold colloid (2.58 × 10⁻¹³ mol, pH6.5) was added, followed by 3 hours' violent stirring at room temperature. Centrifuging the systems [42,000 g (g is acceleration gravity), 30 minutes], the solution parts were removed and 3 mL each of THF was added to the residues and ultrasonically re-dispersed.
Characteristics analysis of these samples was conducted using UV

In this preparation of gold particles using said polymers, it was confirmed that the UV spectrum of the unmodified gold particles showed a large absorption peak at not less than 600 nm, which peak being attributable to the particle aggregation, from the UV-vis spectrum taken of the re-dispersion in the THF solution after the centrifugation. The gold particles treated with acetal-PEO-OH (Control) did not have a large peak at 600 nm or more, like the UV spectrum of the unmodified gold particles, but it was confirmed as a whole that the peaks shifted to higher wavelength side and stability of the fine particulate dispersion was more or less impaired. On the other hand, when the residues after the centrifugation were re-dispersed in a pH3 aqueous solution, acetal-PEG-SH alone was very stable and its re-dispersibility after lyophilization with benzene was also confirmed to be good.

### (3) Characteristic properties of PEG-modified gold fine particles (zeta potential)

With dispersion systems of ordinary gold fine particles in aqueous solutions of, particle surfaces are negatively charged to stabilize the dispersion by the charge repulsion. Whereas, with PEG-modified gold fine particles, complete absence of any charge on their surfaces was confirmed by their zeta potential measurement (Otsuka Electronics: ELS 8000). That is, while commercially available gold fine particles had a zeta potential of -34.5 m V, that of the gold fine particles-hetero PEG conjugate (acetal-PEG-SH/Au) which we prepared this time was -0.86 m V, indicating substantial absence of any charge within the error range at the particle surfaces, i.e., that the surfaces were covered with PEG chains.

The measured data are shown in Table 1.

**TABLE 1**

| Zeta Potential of Acetal-PEG-SH/Gold (AU) Particles | |
|---|---|
| Sample | Zeta Potential (mV) |
| unmodified gold particles | -34.5 |
| acetal-PEG-SH/Au | -0.86 (average value of 3 measurements) |

### Solutions measured:

Phosphate buffer solution to which total 10mM (molar concentration) of buffers NaH₂PO₄ · 2H₂O plus Na₂HPO₄ · 12H₂O was added to adjust its ionic strength to 0.015 and its pH, to 7.5.

Measuring equipment: ELS-8000 (Otsuka Electronics)

### Production Example 3:

Preparation of PEG-modified gold fine particles (3)
In this Example, polyethylene glycolated CdS semiconductor fine particles were prepared using an (acetal-PEG-PAMA) polymer of the formula, 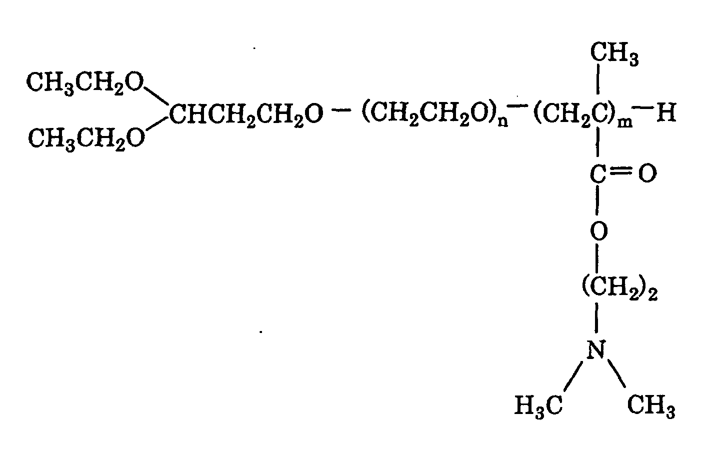 (which was obtained according to the method described in said Kataoka et al., Macromolecules, 1999, 32, 6892 - 6894, in which Mw of PEG was 5,000 g/mol; n and m of PAMA (poly[(2-N, N-dimethylamino) ethyl methacrylate]) were 130 and 100, respectively. One (1) mL of 2.5 mg/mL chloroauric acid (HAuCl₄) aqueous solution and 5 mL of 6 mg/mL acetal-PEG/PAMA block copolymer aqueous solution (NH:Au=8:1) were mixed and stirred at room temperature for 24 hours. At every prescribed time passage UV-vis spectrum of the system was taken, whereby it was confirmed that 540 nm peak attributable to the gold fine particles gradually rose to indicate production of a colloidal particles' (fine particles') dispersion with no reducing agent added. This solution was measured by means of light scattering (DLS: Dynamic Light Scattering) to confirm formation of mono-dispersed colloidal particles of 12 nm in average particle size.

Formation of perfectly uniform particles was further confirmed with transmission electron microscope. When pH of this solution was varied within a range of 2 - 10 and let stand for a day, no change occurred in its spectrum, verifying that very stable gold colloidal particles (fine particles) were obtained in this system.

To this solution, ten equivalent times of the block copolymer of 1,2-diamino-4,5-dimethoxybichloride (DDB) was added, and the pH of resulting solution was adjusted to 2.45 with NaCl. The solution was dyalyzed with a dialisys membrane having a molecular weight cut off of 500, and subjected to a fluorescent analysis at an excitation wavelength of 269 nm. Strong fluorescence was observed at 410 nm, whereby it was confirmed that the terminal acetal groups of the acetal-PEG/PAMA block copolymer on surfaces of the formed gold particles were converted to aldehyde groups and effectively reacted with DDB. Various functional moieties can be bound via so formed aldehyde groups.

### Production Example 4:

Preparation of PEG-modified semiconductor fine particles:
Into 80 mL of distilled water, aforesaid acetal-PEG/PAMA block copolymer (4.19 × 10⁻⁷ mol), CdCl₂(6 × 10⁻⁶ mol) and Na₂S · 9H₂O (6 × 10⁻⁶ mol) were added, and stirred for 20 minutes with a stirrer (750 rpm). Thus obtained PEG-modified semiconductor (CdS) fine particles (particle size: 4 nm) were given a fluorescence measurement at an excitation wavelength of 300 nm. Strong fluorescence characteristic of CdS fine particles appeared.

### Example 1: Immobilization of PEG-modified fine particles onto a sensor chip surface:

(1) Preparation of a sensor chip surface
   An ethanol solution of a mixture of 1 mM of N-succinimidyl-3-(2-pyridylthio) propionate (SPDP) and 2 mM of dithiothreitol (DTT) which is SPDP's disulfide bond reducing agent, was reacted with the golden surface of a sensor chip for 2 hours. Thereafter the washed golden surface was immersed in this solution for 30 minutes and further a 0.1 mg/mL streptavidin PBS solution (pH6.4) was let flow over the same surface for 20 minutes to effect streptavidin-modification of the golden surface.
(2) Preparing PEG (acetal-PEG-SH)-modified gold fine particles according to above Production Example 2, pH of the gold fine particle solution was adjusted to 2. Carrying out deprotection of the acetal groups for 2 hours, said groups were converted to aldehyde groups. After adjusting pH of the solution to 6, 4 times the amount of the PEG of biocytin hydrazide was added and reacted for 6 hours under stirring. (Here the "4 times the amount of the PEG" was calculated as follows: the surface area of a gold fine particle was calculated from its particle size, and the surface density of PEG was hypothesized to be 0.25 - 0.40 PEG chain/nm², which is a value calculated from the number of fine particles. Normally the surface density used is 0.25, which was calculated from Tg of the PEGylated gold fine particles). Then NaBH₄ was added, followed by 3 days' stirring. After purification by centrifuge, a solvent-substitued solution thereof was prepared with 10 mM-PBS (pH6.4). The chip having the golden surface as prepared in the above step (1) was immersed in so obtained biotinylated PEG modified gold fine particle solution and the PEG-modified gold fine particles were immobilized on said chip surface.
(3) Characteristics of the surface
   The surface onto which the PEG-modified gold fine particles were immobilized, as prepared in (2) above, was dipped in a 0.1 mg/mL bovine serum albumin (BSA) solution in PBS (pH6.4) for an hour, and the BSA adsorbed onto said surface was quantified by means of SPR. According to the results, BSA adsorption onto the untreated golden surface was, in terms of SPR angle shift, Δθ=0.21°, while that onto the PEG-modified gold fine particles-immobilized surface was: Δθ==0.02°. These data demonstrate that the PEG-modified gold fine particles-immobilized surface inhibits non-specific adsorption of protein BSA in the blood.

### Example 2: Assay by SPR utilizing PEG-modified gold nanoparticles:

In the subsequent descriptions, the flow rate of SPR was always 10 µL/min., and the set temperature was 25°C. Also in all cases the buffers used were advancedly passed through a 0.22 µm filter and thereafter deaerated. An other flow path on which no lectin was immobilized was provided for control. Also as the regenerating solution to dissociate all of the gold nanoparticles bound to the lectin on the sensor chip surface, a buffer containing 100 mg/mL of galactose was used.

### A) Immobilization of lectin on the sensor chip

An SPR sensor chip (CM5: purchased from BIACORE) was inserted in a flow path provided on the SPR sensor chip surface and through which phosphate buffer solutions (pH7.4 and 10.15, respectively) were let flow until stabilization. Then 100 µL of a 1:1 mixed solution of EDC (N-ethyl-N'-(3-dimethylaminopropyl)-carbodiimide hydrochloride) and NHS (N-hydroxysuccinimide) was injected into the flow path to activate carboxyl groups on the chip. In succession, RCA₁₂₀ (50 µg/mL, the solvent was an acetate buffer solution of pH5.0) was injected for immobilization, and finally 70 µL of 1M ethanolamide hydrochloride was injected to block the remaining activated NHS groups. Calculated from the difference between the RU measured then and the initial RU, the quantity of immobilized lectin was 5600 RU (^{∼} 5.6 ng/mm²= 2.8 × 10⁻² lectin/nm²).

### B) Confirmation of specific binding of lac 65 and lectin [lac 65 meaning a sample having lactose on 65% of the PEG terminals of the PEG (PEG chain number: 520) on the gold nanoparticles]

### Test method

The binding between the lectin on the sensor chip surface and the gold nanoparticles was measured by injecting 40 µg/mL of lac65 into the flow path for 1800 seconds (300 µL). Thereafter said buffer was let flow for 3,000 seconds and dissociation of gold nanoparticles was measured. Finally, 100 mg/mL of galactose was injected to regenerate the sensor chip.

### Result and observations

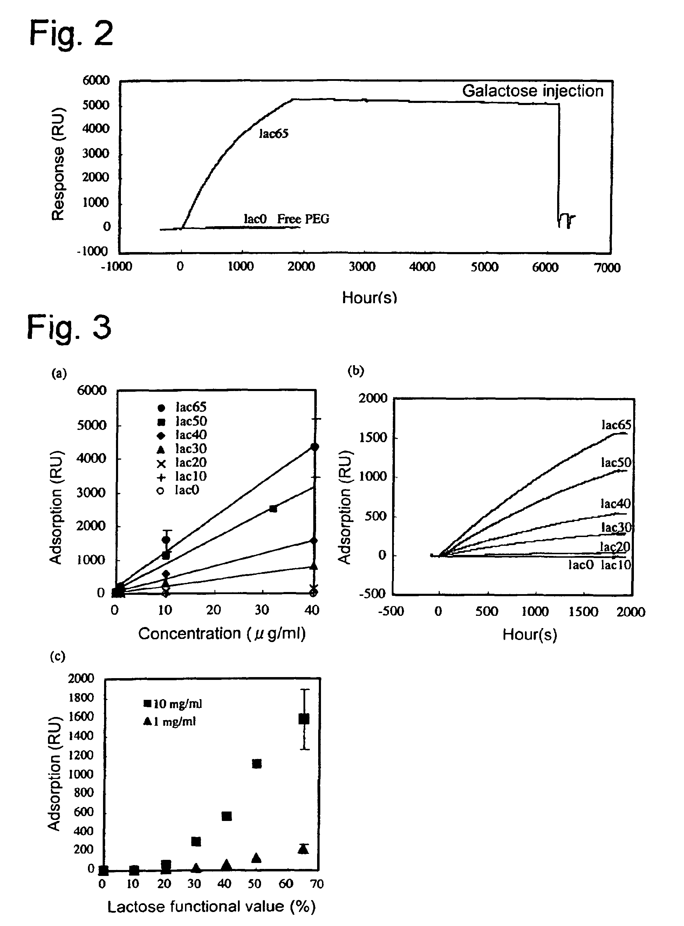

The sensorgram of lac 65 is shown as Fig. 2. While free lactose-PEG-S-S-PEG-lactose at the same concentration showed nearly no RU increase, lac 65 caused an increase of 5200 RU. Also when compared with a micelle system having lactose at the surface, while injection of 500 µg/mL of the micelle into the flow path on the sensor chip surface onto which 7,000 RU of lectin was bound obtained ^{∼}1,400 RU response, the gold nanoparticles used in this experiment, which had approximately the same particle diameter to that of this micelle, produced 4-fold response at a concentration of 1/10 that of the micelle. Hence, it was verified that the response was markedly increased by the presence of the gold nanoparticles. The reasons for achieving such a high response are considered to be, first, the very great specific gravity of gold nanoparticles substantially raised the dielectric constant at the chip surface, and also the surface plasmon interaction between the gold substrate on the chip and the gold nanoparticles.

The fact that the flowing of the buffer caused almost no dissociation of the gold nanoparticles showed that the binding of the gold nanoparticles to the lectin was very strong.

While the binding could not be confirmed with lac 0, it was confirmed as to lac 65 and the addition of galactose was confirmed to largely decrease the response, i.e., to cause dissociation of gold nanoparticles. These facts suggest specific binding of the lactose at the surface layer of the gold nanoparticles and RCA 120 lectin.

### C) Effect of ligand density in the binding

### Test method

Three-hundred (300) µL each of gold nanoparticles solutions with 0 - 65% lactose density at the surfaces (lac 0, lac 10, lac 20, lac 30, lac 40, lac 50, lac 65) at varied concentration levels of 40, 10, 1, and 0.1 µg/mL were injected and the quantities of the lactose binding were measured.

### Results and observations

First, the correlation between the concentration and response of lac 0 - lac 65 is shown in Fig. 3 (a) which shows the result the higher the lactose density and the higher the gold nanoparticle concentration, the more RU increased. Lac 0 - lac 65 sensorgram where the gold nanoparticle solutions of each 10 µg/ml concentration were injected is shown in Fig. 3 (b), and the relationship between the ligand density and bound quantity (RU) is shown in Fig. 3 (c). From the results shown by those graphs, it is understood that hardly any lectin-lactose binding occurred with lac 10, a little binding was confirmed with lac 20, and at higher ligand densities the binding was promoted with the density increase. This approximately coincides with the results of the UV analyses. In our observation of the UV experiment results, we thought the reasons for this critical value of 20% were the following three: 1) excessive presence of lectin in the solution caused capping of the particles with the lectin, 2) for detecting surface plasmon as a change in the spectrum, aggregation of the gold nanoparticles beyond a certain extent was necessary, and 3) polyvalent binding. Because capping does not take place with SPR sensor chip, and a spectral change of surface pasmon is not observed as in the case of UV. Hence the reason for the critical value is considered to be polyvalent binding. Specifically, it is presumed that a high ligand density causes binding of many ligands with lectin, forming strong bonds. Whereas at low ligand densities only a minor number of ligands could participate in the binding, like 1:1 ligand-lectin binding.

### D) Effect of ligand density in dissociation

### Test method

Several tens µL each of solutions of gold nanoparticle with 30 - 65% lactose density at their surfaces (lac 30, lac 40, lac 50, lac 65) at a concentration of 40 µg/mL was injected to cause binding of about 400 RU of the gold nanoparticles. Then buffer solutions each containing 0.1 µg/mL or 1 µg/mL of galactose was injected to quantify the dissociation at each of the galactose concentration.

### Results and observations

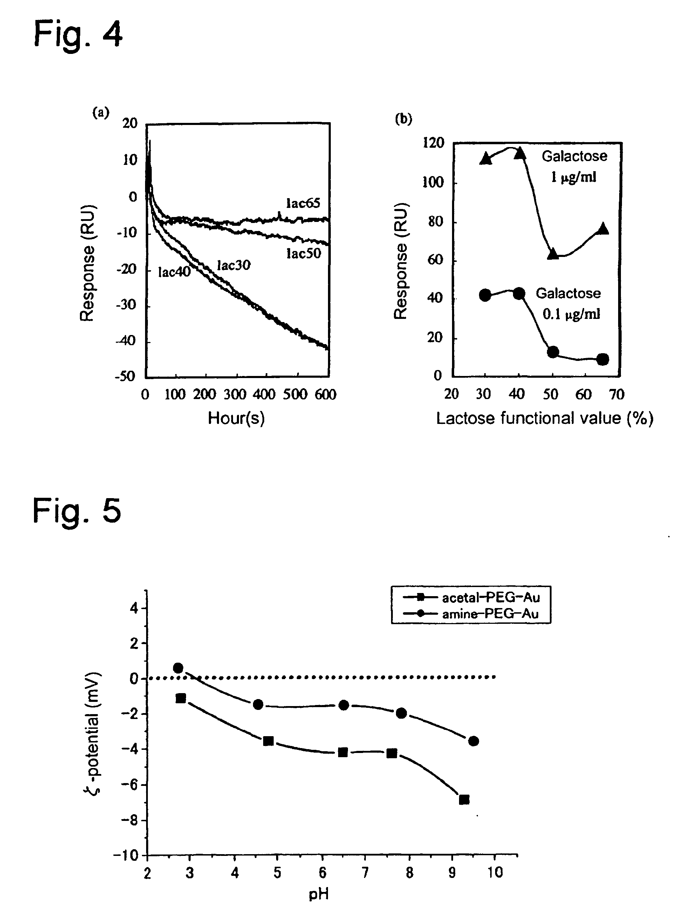

Fig.4 (a) shows the sensorgram obtained when 0.1 µg/mL of galactose was injected, whereby gradually occurring dissociation of lac 30 and lac 40 with time was confirmed, but nearly no dissociation of lac 65 and lac 50 was observed. The relationship between the ligand density and dissociation quantity was as shown in Fig. 4 (b), in which distinct difference was observed between 40% and 50%. That is, compared with the dissociation quantities of lac 30 and lac 40, those of lac 50 and lac 65 were considerably less. This is considered to be relevant to the large difference in the increase in NIA between the ligand density of 40% and 50% in the UV experiment. We infer that presumably at a certain point between said ligand densities the valance number in polyvalent binding changed.

Furthermore, it was demonstrated, where high sensitivity detection of the analyte through the dissociation was aimed at, down to no more than 0.1 µg/mL could be detected when the ligand density was not higher than 30%. By contrast, when the ligand density was 50% or higher, the detection limit concentration became higher. In respect of the dissociation, therefore, moderately lower ligand density contributes to increase the detection sensitivity. Hence, minute studies of the effect of ligand density will open prospects for versatile applications.

### Production Example 5: Preparation of unbound end-aminated particles

A commercial solution of gold fine particles (5 nm) was mixed with 5 × 10⁴ times its amount of a reaction solution resulted from adding NaBH₄ as a reducing agent to acetal-PEG-SH to allow their reaction for an hour and then adjusting the pH to 6.5, the same pH value to that of the gold fine particle solution, and the mixture was reacted for an hour under stirring. Then 1 mg/mL of HO-PEG-OH was added to the system, followed by 2 hours' reaction in a water bath which was maintained at 75°C. The excessive polymer was removed from this stabilized gold colloidal solution (gold fine particle size: 5 nm, PEG 4500) by centrifuge (4°C, 350,000 × g, 40 min.), the solution pH was adjusted to 2 with HCl and the acetal groups were deprotected (2 hours). After the deprotection the pH was raised to 6 with NaOH, to which ammonium acetate was added under the conditions as shown in the following Table 2, followed by 3 hours' reaction. After said 3 hours' reaction, the respective reducing agent was added to the PEG and stirred. Twenty-four hours thereafter, each system was purified by centrifuge (4°C, 350,000 × g. 30 minutes) and the residues were re-dispersed in ultrapure water. For confirming the end-amination, zeta potential was measured.

**TABLE 2**

| Amination Reaction Conditions | | | |
|---|---|---|---|
| Run No. | Ammonium Acetate (mg/mL) | Reducing agent | Addition method of reducing agent |
| 1 | 10 | triacetate | Ten times the PEG quantity of the reducing agent was added 3 times at 2 hours' intervals, followed by a day's stirring. |
| 2 | 10 | sodium borohydride | Ten times the PEG quantity of the reducing agent was added, followed by a day's stirring. |

The measured result was as shown in Fig. 5, which confirmed the amination, by the zeta potential becoming positive at the low pH range.

### Example 3: Method of directly immobilizing gold nanoparticles on SPR chip surface and biotinvlating the PEG terminals:

Ozone-treated (15 minutes with ozone washing machine) gold chips were prepared. The chips were immersed (an overnight) in PEG-OH (2000) solution at a concentration of 1 mg/mL which contained separately prepared PEGylated gold nanoparticles having amino groups (PEG 4500, particle size 5 nm, 0.76 × 10⁻¹⁰ mol/mL) (Sample 1).

The gold chips were mounted on BiaCore 3000, and over which sulfosuccinimidyl-D-biotin (0.1 mg/mL; flow rate, 20 µL/min.) was let flow for 10 minutes to biotinylate amino end groups of the PEG (Sample 2). Then a 10% aqueous solution of acetic anhydride was let flow (flow rate, 10 µL/min.) for 20 minutes to acetylate unreacted amino groups (Sample 3). Thus prepared SPR sensor chip surfaces were subjected to a protein adsorption test.

### Example 4: Method of introducing activated ester onto SPR sensor chip surface utilizing SPDP, for immobilizing PEGylated gold nanoparticles having amino groups:

Ozone-treated (15 minutes with ozone washing machine) gold chips were prepared, which were immersed in an ethanol solution of 1 mM SPDP and 2mM DTT for 30 minutes.

Further the chips were immersed (an overnight) in PEG-OH (2000) solution at a concentration of 1 mg/mL which contained separately prepared aminated gold nanoparticles (PEG 4500, particle size 5 nm, 0.76 × 10⁻¹⁰ mol/mL) (Sample 4).

The gold particles were mounted on BiaCore 3000 and sulfosuccimidyl-D-biotin (0.1 mg/mL, flow rate 20 mL/min.) was let flow thereover for 10 minutes to biotinylate end amino groups of the PEG (Sample 5). Then a 10% aqueous acetic anhydride solution (flow rate, 10 µL/min.) was let flow for 20 minutes to acetylate unreacted amino groups (Sample 6). Thus prepared SPR sensorchip surfaces were subjected to a protein adsorption test.

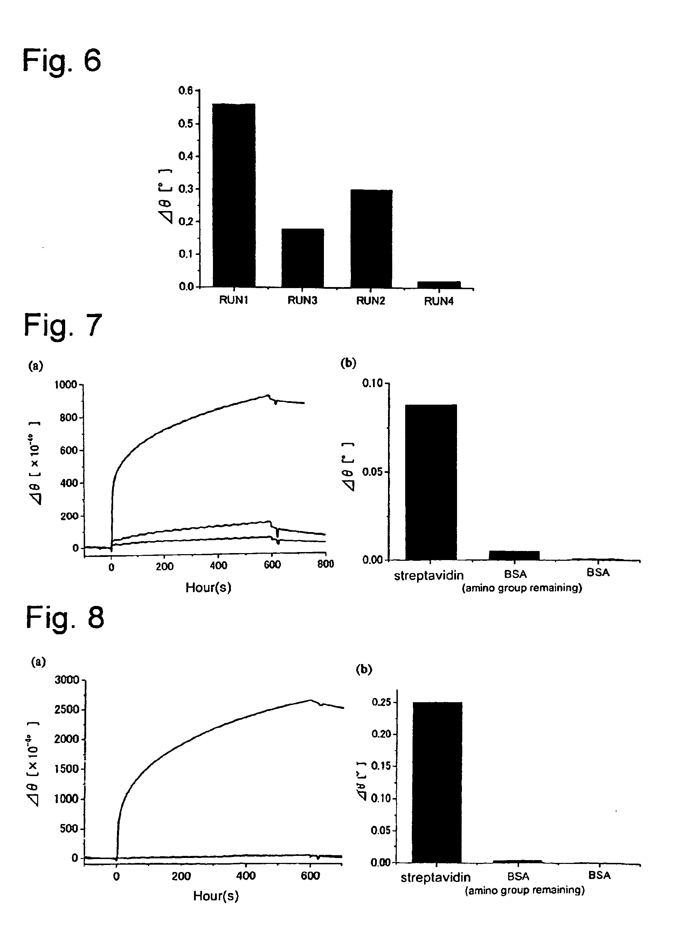

The results were as shown in Fig. 6, in which Run 1 shows the result of using Sample 1; Run 2, that of using the acetal-PEG gold nanoparticles in place of Sample 1; Run 3, that of using Sample 4; and Run 4, that of using the acetal-PEG gold nanoparticles in place of Sample 4. From Fig. 6 it can be understood that the carried amount of the PEGylated gold nanoparticles having amino groups on the SPR sensorchip surface was large.

The status of the response variation at each stage of the surface preparation are shown in the following Table 3.

**TABLE 3**

| Status of Response Variation at Each Stage of Surface Preparation | | |
|---|---|---|
| | Immobilization by direct adsorption 1-(1) (× 10⁻⁴⁰) | Immobilization by SPDP 1-(2) (× 10⁻⁴⁰) |
| Varied amount by SPDP | - | 2800 |
| Varied amount by gold fine particles | 5500 | 2980 |
| Varied amount by active ester biotin | 397 | 949 |
| Varied amount by acetic anhydride | 390 | 158 |

The result of observing specific and non-specific adsorption onto the surface (1) by direct adsorption, i.e., the surface prepared by directly binding aminated gold fine particles to the gold chip surface by immersion, was as shown in Fig. 7. In Fig. 7, the uppermost line represents adsorption of streptavidin; the middle line, that of BSA (with amino groups still remaining); and the lowest line, that of BSA. The bar graph shows the results of, from the left, adsorbing streptavidin onto Sample 1, BSA onto Sample 2, and BSA onto Sample 3.

The result of observing specific and non-specific adsorption onto the surface (2) formed by immobilizing aminated gold fine particles on gold chip surface utilizing SPDP is shown in Fig. 8.

From Figs. 7 and 8, it can be understood that more of the specific adsorption of streptavidin onto the surface (2) on which the immobilization was mediated by SPDP in Fig. 8 was observed. With heretofore prepared biotinylated gold fine particles-bound surfaces, a large difference sufficient to distinguish specific adsorption from non-specific adsorption was not observed in streptavidin and BSA adsorptions. By contrast, in Fig. 8 a large difference of 2500 (× 10⁻⁴⁰) and 9 (× 10⁻⁴⁰) can be confirmed.

### Industrial Applicability

According to the present invention, high sensitivity sensor systems for bioassays for detecting analytes in biological fluids are provided, in which non-specific adsorption of impurity proteins can be suppressed. This invention is useful, therefore in the trade of manufacturing various diagnostic machinery and tools, and also in the art of diagnosis.

## Claims

1. A biosensor system for bioassay which comprises, as a set,
(A) polyethylene glycol-modified nanoparticles of a structural formula I:
(X-W²-PEG-W¹-L)ₓ-PCL-(L-W¹-PEG-W²-Y)_{y} (I)
in which
PCL stands for a free electron metal fine particle, metal oxide fine particle or semiconductor fine particle;
X stands for a functional group or functional moiety capable of binding to a biosensor chip surface;
Y stands for at least one group or moiety which is selected from the group consisting of C₁-C₆ alkyl, optionally protected functional groups which are useful for forming said functional group or functional moiety X, and functional moieties same as , or different from, X;
L stands for a linker or linkage portion linked to PCL;
W¹ and W² stand for single bonds or same or different linkers,
PEG stands for ethylene oxide units, (-CH₂CH₂O-)ₙ (wherein n is an integer of 5 - 10,000),
W²-PEG-W¹-L in (X-W²-PEG-W¹-L)ₓ and
(L-W¹-PEG-W²-Y)_{y} may be same or different, and
x and y are integers not less than 1 independently of each other, which together represent an integer sufficient for the PEG chains to cover the PCL surface in an aqueous medium and
(B) a biosensor chip having a surface to which above (A) particles can bind via X and which surface is made of glass or a material corresponding to that of PCL.

2. A biosensor system according to Claim 1, in which said (A) particles are carried on one surface of the (B) biosensor chip as the particles are linked to the biosensor chip surface via X, to substantially cover a part or whole area of said surface.

3. The biosensor system according to Claim 1, in which said (A) particles and (B) biosensor chip surface are used in a state of either being capable of binding to each other or being bound, the binding being such that can be replaced by an analyte in an aqueous medium due to competitive action of the analyte.

4. A biosensor system according to Claim 1, in which -L- in the structural formula I is a group selected from a group consisting of 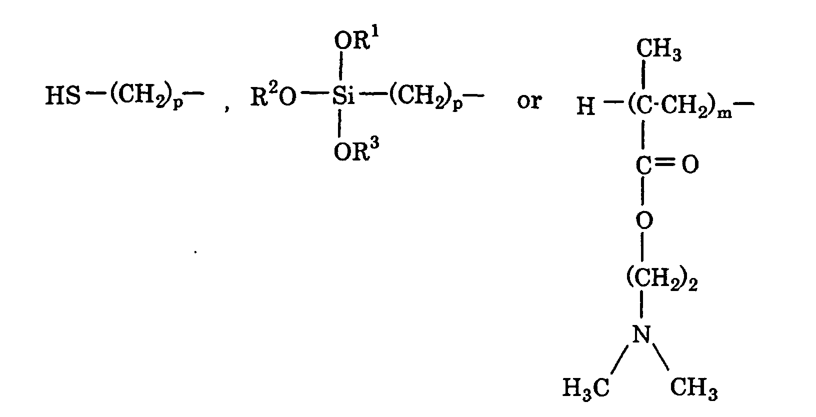 (in which p is independently an integer of 2 - 12, R¹, R² and R³ each independently stands for C₁-C₆ alkyl, and m is an integer of 2 - 100); and
W¹ and W² each independently stands for a group selected from the group consisting of single bond, C₁-C₆ alkylene, -COO- (binding to methylene group in ethylene oxide unit via oxygen atom), -O-, -S-, -( C₁-C₆ alkylene) -COO-, -( C₁-C₆ alkylene) -O- and -(C₁-C₆ alkylene) -S-.

5. A biosensor system according to Claim 1, in which X in the structural formula I representing said (A) particle is a residue of a member forming a biological specific binding pair; and (B) sensor chip has a thin membrane surface made of a material corresponding to that constituting PCL in the structural formula I, said surface carrying the other member which forms said biological specific binding pair with said member X, either directly or via at least one of C₁-C₆ alkylene or (-CH₂CH₂O-)ₙ (wherein n is an integer of 5 - 10,000).

6. A biosensor system according to Claim 1, in which X in the structural formula I representing said (A) particle stands for any one of the following groups 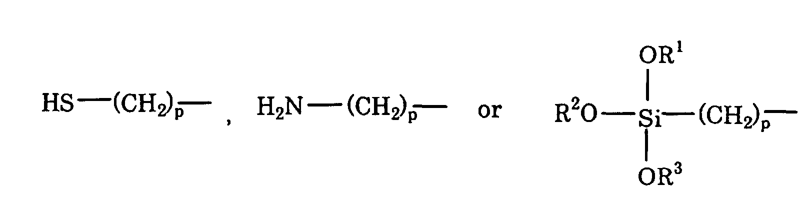 (in which p is an integer of 2 - 12 independently of each other; R¹, R² and R³ each independently stands for C₁-C₆ alkyl); (B) sensor chip has a thin membrane surface made of any one of the materials forming PCL of the structural formula I or a glass surface; and said (A) particles and surface of (B) sensor chip are linked to each other via the functional group X, X having trialkoxysilyl where surface of (B) is made of glass.

7. A biosensor system according to Claim 5, in which Y in the structural formula I representing said (A) particles is a group selected from those of the following formulae: 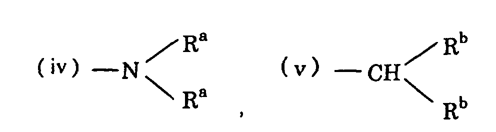 or
(vi) ―COOH
(in which R^{a} each independently stands for hydrogen or C₁-C₆ alkyl; R^{b} each independently stands for a C₁-C₆ alkyloxy; or the two R^{b}'s together stand for an atomic group forming oxy or an optionally C₁-C₆ alkyl-substituted ethylene group).

8. A biosensor system according to Claim 1, in which x + y in the structural formula I representing said (A) particles is an integer corresponding to 0.1 - 0.5 per 1 nm².

9. A biosensor system according to Claim 1, in which PCL in said (A) particle has an average cross-sectional length of 5 - 500 nm.

10. A polyethylene glycol-modified nanoparticle of a structural formula I
(X-W²-PEG-W¹-L)ₓ-PCL-(L-W¹-PEG-W²-Y)_{y} (I)
in which
PCL stands for a free electron metal fine particle, metal oxide fine particle or semiconductor fine particle;
X stands for a functional group or functional moiety capable of binding to a biosensor chip surface;
Y stands for at least one group or moiety which is selected from the group consisting of C₁-C₆ alkyl, optionally protected functional groups which are useful for forming said functional group or functional moiety X, and functional moieties same as , or different from, X;
L stands for a linker or linkage portion linked to PCL;
W¹ and W² stand for single bonds or same or different linkers,
PEG stands for ethylene oxide units, (-CH₂CH₂O-)ₙ (wherein n is an integer of 5 - 10,000),
W²-FEG-W¹-L in (X-W²-PEG-W¹-L)ₓ and
(L-W¹-PEG-W²-Y)_{y} may be same or different, X being a residue of a member to form a biological specific binding pair, Y being a group other than the residue of the member forming said biological specific binding pair, L standing for a group of the formula 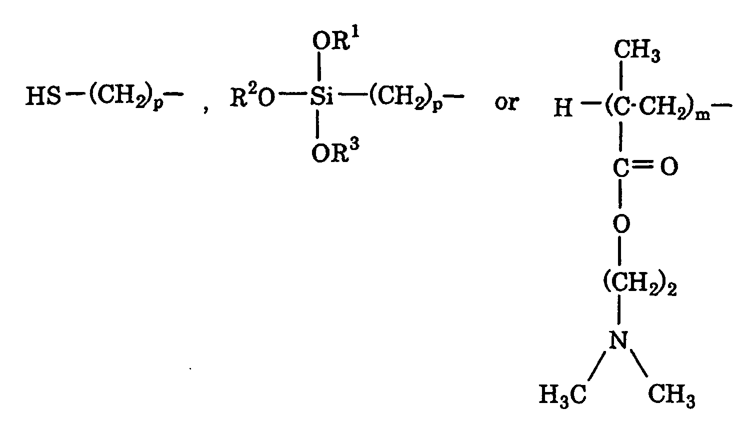 (in which p is an integer of 2 - 12, R¹, R² and R³ each independently stands for C₁-C₆ alkyl, and m is an integer of 2 - 100);
x + y is a number corresponding to 0.1- 0.5 per 1 nm² of the PCL surface, (x/ x + y) × 100 being an integer of 1 - 99, and the average dimension of cross-section of the PCL is 5 - 500 nm.

11. A polyethylene glycolated nanoparticle according to Claim 10, in which said member to form a biological specific binding pair is a residue derived from a substance selected from a group consisting of monosaccharide or oligosaccharide, antigen or hapten, substrate, hormone and oligonucleotide.

12. A method of detecting an analyte in a biological fluid, which comprises:
(a) preparing polyethylene glycol-modified nanoparticles as described in Claim 10,
(b) preparing a biosensor chip having a thin membrane surface made of a material corresponding to that forming PCL of the nanoparticles, said surface carrying, either directly or via at least a C₁-C₆ alkylene or (-CH₂CH₂O-)ₙ (wherein n is an integer of 5 - 10,000), a member which is to form a biological specific binding pair with the other member present in X of said nanoparticles,
(c) contacting said particles (a) and biosensor chip (b) with a biological fluid which is suspected to contain either one of the members capable of forming the biological specific binding pair as an analyte,
(d) determining the change in the extent of linkage of the particles (a) to the biosensor chip (b) surface caused by the competitive action of the analyte and
(e) using the change as an index of the analyte concentration in said biological fluid.

13. A detection method according to Claim 12, in which the change in the extent of linkage of the particles (a) to the biosensor chip (b) surface in the step (d) is detected as a change in surface plasmon resonance spectrum.

14. A detection method according to Claim 12, in which the pair formed by two members capable of forming a biological specific binding pair is selected from the group consisting of sugar - lectin, antigen or hapten - antibody, substrate - enzyme, hormone - receptor protein, oligonucleotide - either oligonucleotide or polynucleotide which contain complementary chain sequence of the first oligonucleotide.

15. A detection method according to Claim 12, in which said particles (a) and the biosensor chip (b) surface form biological specific binding pairs and are linked in advance.
